# EUROPEAN PATENT SPECIFICATION

(11) **EP 0 761 613 B1**
(45) Date of publication and mention of the grant of the patent: **24.10.2001**
(21) Application number: 96306030.6
(22) Date of filing: 19.08.1996
(51) Int. Cl.: C03B 29/00, C03B 29/08, C03B 23/033, C03B 27/012, C03B 27/044

(54) **Method for heating and forming a glass sheet**
Verfahren zum Erhitzen und Formen von Glastafeln
Procédé de chauffage et de formage d'une feuille de verre

(30) Priority: 07.09.1995 US 524495
(43) Date of publication of application: 12.03.1997
(73) Proprietor: Ford Motor Company, Dearborn, MI 48126 (US)
(72) Inventor: Boaz, Premakaran Tucker, Livonia, Michigan 48154 (US)
(74) Representative: Messulam, Alec Moses

(56) References cited:
- EP-A- 0 000 269
- EP-A- 0 546 617
- WO-A-93/06052
- DE-C- 686 549
- DE-C- 707 141
- US-A- 3 545 951
- US-A- 3 938 980
- US-A- 4 065 284
- US-A- 4 100 386

## Description

The present invention relates generally to glass sheets and, more specifically, to a method for heating and forming a glass sheet.

It is known to heat glass sheets using a "hearth" or "lehr". Generally, the lehr is a furnace and may be of a continuous roller-type, fixtured roller-type or gas-type. For example, a roller-type lehr has a plurality of rollers disposed beneath a plurality of radiant heaters. Typically, a glass sheet is placed inside the lehr where it is heated by conventional radiation, convection and conduction heat. The glass sheet is moved along the rollers at a predetermined rate which depends on the thermal conductivity of the glass sheet to reach a temperature in its forming range. When the glass sheet is at a temperature in its forming range, the heated glass sheet is formed to a predetermined configuration using a plurality of forming rollers to bend the glass sheet to a desired curvature. Once formed, the glass sheet is either quenched, annealed or tempered.

Although the above lehr and forming rollers have worked well to heat and form a glass sheet, they suffer from the disadvantage that the lehr must be long in length to allow the glass sheet to be heated at the predetermined rate. They also suffer from the disadvantage that the process capability is limited due to heating the glass sheet with only radiant heat. They further suffer from the disadvantage that the forming rollers are limited to a single radius capability at a time. As a result, there is a need in the art to heat a glass sheet quickly in a controlled manner and to form the glass to a predetermined configuration using forming rollers.

US-A-3,938,980 discloses a method of tempering an elongated glass article having a regular, non-planar cross section defining interior and exterior main surfaces and having adjacent edge portions, without significant deformation thereof. The method comprises the steps of supporting the weight of the article on the edge portions, subjecting at least transverse increments of the article to a source of homogeneous heat and simultaneously quenching the surfaces while the article is supported on the edge portions. In this way, there is defined within the article a temperature differential condition in which interior portions of the article are at temperatures at least as high as the annealing temperature of the glass while the surfaces of the glass are at a temperature sufficiently below the annealing temperature to remain rigid and, hence, susceptible of supporting the weight of said article without deformation under gravitational influences.

US-A-3545951 discloses a method of shaping essentially flat sheets, in particular glass sheets, into curved form, comprising, mounting a plurality of like arcuate forms for pivoting, each about its own chordal axis, all the axes being parallel and spaced in a common plane, pivoting all the forms into the common plane, depositing a sheet in plastic condition onto the forms for support and shaping conjointly thereby, and pivoting the forms in unison, each about its own chordal axis, until they conjointly define a surface of desired curvature.

Accordingly, the present invention is a method for heating and forming a glass sheet. The method includes the steps of heating a glass sheet to at least a first predetermined temperature and applying microwave energy to the glass sheet to heat the glass sheet to at least a second predetermined temperature. The method also includes the steps of cooling an outer surface of the glass sheet to at least a third predetermined temperature and forming the glass sheet using forming rollers to a predetermined configuration.

One advantage of the preferred embodiment of the present invention is that an improved method is provided for heating and forming a glass sheet. Another advantage of the present invention is that the method uses microwave energy to heat the glass sheet quickly, preferably to a temperature at or above its softening point. An advantage of a preferred embodiment of the present invention is that the method uses pinch rollers to form the glass sheet to a desired curvature. Another advantage achieved by a preferred embodiment of the present invention is that the method uses a series of sleeved pinch rollers of different radii to form the glass sheet to a desired curvature. A further advantage of the present invention is that the method allows for heating and cooling of the glass sheet at the same time. Yet a further advantage of the present invention is that the method reduces the length of the lehr, resulting in less floor space and increased throughput (speed and yield) of glass sheets formed.

The invention will now be described, by way of example, with reference to the accompanying drawings, in which:
FIG. 1 is a fragmentary perspective view of a lehr used in conjunction with a method for heating and forming a glass sheet, according to the present invention;
FIG. 2 is a sectional view taken along line 2-2 of FIG. 1;
FIG. 3 is a sectional view taken along line 3-3 of FIG. 1;
FIG. 4 is a sectional view taken along line 4-4 of FIG. 1; and
FIG. 5 is a graph of temperature versus rate of a glass sheet heated by a method for heating and forming a glass sheet, according to the present invention.

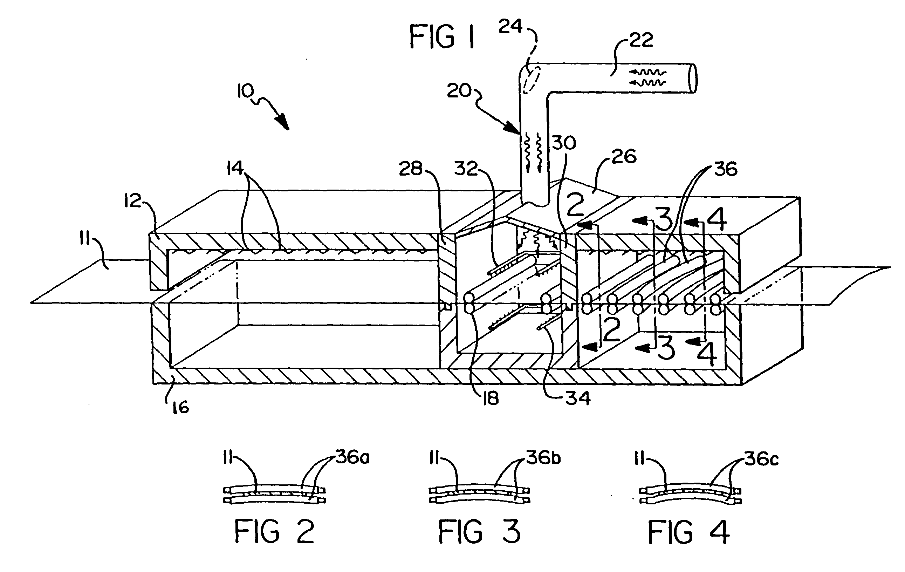

Referring to the drawings and in particular to FIG. 1, one embodiment of a lehr 10 for use in conjunction with a method for heating and forming a glass templet or sheet 11, according to the present invention, is shown. As illustrated, the lehr 10 is of a roller-type continuous furnace.

The lehr 10 includes an upper housing 12 extending longitudinally and having a plurality of heaters 14 spaced longitudinally therealong. The heaters 14 are of the radiant type as is known in the art. The lehr 10 also includes a lower housing 16 extending longitudinally and having a plurality of rollers 18 disposed longitudinally therealong. It should be appreciated that the glass sheet 11 is moved by the rollers 18 as is known in the art. It should also be appreciated that, up to this point in the description, the lehr 10 is conventional and known in the art.

Referring to FIG. 1, the lehr 10 includes a microwave energy apparatus, generally indicated at 20, disposed at a position along the length of the lehr 10. The microwave energy apparatus 20, partially shown, includes a conduit 22 extending longitudinally and having a reflector 24 in a corner thereof to direct microwave energy through a downward portion of the conduit 22. The microwave energy apparatus 20 includes a shield 26 at one end of the conduit 22 to columnate the microwave energy and form a transverse curtain of microwave energy (e.g. 15 cms (six inches)) toward the rollers 18. The microwave energy apparatus 20 is a self-contained unit having a microwave energy frequency of two (2) to forty (40) gigahertz. Preferably, the frequency of the microwave energy is less than thirty-six (36) gigahertz. The lehr 10 includes uplift doors 28 and 30 on each longitudinal end of the shield 26 to allow entry and exit of the glass sheet 11 into a contained area for the microwave energy. The lehr 10 also includes air blowers 32 and 34 above and below the rollers 18 and disposed in the area between the uplift doors 28 and 30. The air blowers 32 and 34 are arranged in two rows to direct cooling air toward the glass sheet 11. It should be appreciated that the microwave energy apparatus 20 is a Gyrotron type commercially available from Continental Electronics of Dallas, Texas. It should further be appreciated that a temperature measuring device is used to measure the temperature of the glass sheet 11 which is conventional and known in the art.

The lehr 10 further includes a plurality of forming rollers 36 for forming the heated glass sheet 11 to a predetermined configuration. The forming rollers 36 are sleeved pinch rollers as is known in the art. As illustrated in FIGS. 2 through 4, the forming rollers 36 include a plurality of first radius rollers 36a, second radius rollers 36b and third radius rollers 36c having a curvature of varying degree to form the glass sheet 11 to a predetermined curvature as is known in the art. For example, the first radius rollers 36a have a 274 cms (108 inch) inch radius, the second radius rollers 36b have a 229 cms (90 inch) radius and the third radius rollers 36c have a 127 cms (850 inch) inch radius. It should be appreciated that in one pair of rollers 36, one roller is fixed and the other roller is adjusted relative to it. It should also be appreciated that the smallest radius forming roller 36 is farthest downstream.

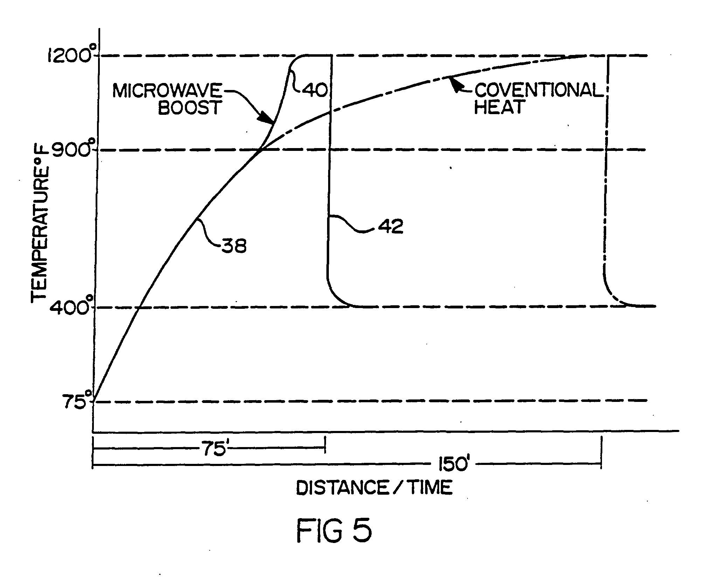

In operation, the lehr 10 may be used to form the glass sheet 11 as a windshield or door glass for a motor vehicle (not shown) by a method, according to the present invention. The method includes placing a flat or planar glass sheet 11 on the rollers 18 at one end of the lehr 10. The method includes moving the glass sheet 11 along the rollers 18 at a predetermined rate and heating the glass sheet 11 to a predetermined temperature with the heaters 14. For example, the glass sheet 11 is heated by the heaters 14 using an ambient heat of over 760°C (1400°F) as the glass sheet 11 travels a certain distance over time to heat the glass sheet 11 to a predetermined temperature. In one embodiment, the predetermined temperature is the softening point of the glass sheet 11 which is approximately 482 to 510°C (900°F to 950°F). As illustrated in FIG. 5, a curve 38 represents the temperature of the glass sheet 11 as it moves over distance/time through the lehr 10 as is known in the art.

When the glass sheet 11 is at its softening point, the glass sheet 11 is disposed between the uplift doors 28 and 30. The method includes applying microwave energy to the glass sheet 11 when the glass sheet 11 is at or above its softening point as represented by the curve 40 as illustrated in FIG. 5. The method includes moving the glass sheet 11 at a predetermined rate under the curtain of the microwave energy columnated by the shield 26 and rapidly heating the glass sheet 11 with the microwave energy to a predetermined temperature. In one embodiment, the predetermined temperature of the glass sheet 11 is its forming range of approximately 621 to 676°C (1150°F to 1250°F). For example, the temperature of the glass sheet 11 can be raised from 482°C (900°F) to over 649°C (1200°F) in less than ten (10) seconds as illustrated in FIG. 5. The microwave energy heats the glass sheet 11 directly by generating heat at the molecular level by creating polar orientation movement very rapidly resulting in instantaneous and uniform heating through the thickness of the glass sheet 11. The method also includes cooling an outer surface of the glass sheet 11 to at least a third predetermined temperature of approximately 482°C (900°F) simultaneously while applying the microwave energy. For example, air is blown at the glass sheet 11 by the air blowers 32 and 34 to give the glass sheet 11 a cool hard skin such that the outer surfaces of the glass sheet 11 will not be marked by the forming rollers 36 while the inside of the glass sheet 11 is in its forming range. It should be appreciated that if the glass sheet 11 is below its softening point, the microwave energy may break the glass sheet 11. It should also be appreciated that microwave frequencies as low as two gigahertz can be used when the glass sheet 11 is above 482°C (900°F). It should be appreciated that the predetermined rate is based on the intensity of the microwave energy and the coupling rate of the glass sheet 11. It should further be appreciated that the ambient temperature of the lehr 10 before the uplift door 28 is approximately 649°C (1200°F) and the ambient temperature of the lehr 10 after the uplift door 30 is approximately 676°C (1250°F) to maintain the glass sheet 11 at its forming temperature.

When the glass sheet 11 passes beyond the uplift door 30, the method includes forming the glass sheet 11 to a predetermined configuration or curvature. The glass sheet 11 passes through the forming rollers 36a which begin to bend the glass sheet 11 to a large radius curvature and passes through the forming rollers 36b and 36c to bend the glass sheet 11 to achieve smaller radius curvature until reaching the predetermined curvature. The method includes cooling the glass sheet 11 with cool air once the glass sheet 11 exits the lehr 10 to below its softening point as represented by the curve 42 illustrated in FIG. 5.

Accordingly, the method of the present invention provides a way to quickly heat the moving glass sheet 11 by providing a curtain of microwave energy through which the glass sheet 11 is passed inside the lehr 10. The microwave energy is applied based on the size and thickness of the glass sheet 11 to bring the temperature of the glass sheet 11 up to its forming range for the forming process. Further, the method allows the glass sheet 11 to achieve smaller radius by changing from one radius to another by simply employing the proper set of forming rollers 36. The method of the present invention uses the microwave energy curtain to heat the glass sheet 11 and at the same time to cool the outer surface thereof which allows the bending of the glass sheet 11 using a succession of sleeved pinch rollers 36. As illustrated in FIG. 5, the method of the present invention heats the glass sheet 11 to the forming range in less distance/time than conventional radiant heating in a lehr.

## Claims

1. A method for heating and forming a glass sheet, said method comprising the steps of:
heating a glass sheet to at least a first predetermined temperature;
applying microwave energy to the glass sheet to heat the glass sheet to at least a second predetermined temperature;
cooling an outer surface of the glass sheet to at least a third predetermined temperature; and
forming the glass sheet using forming rollers to a predetermined configuration.

2. A method as claimed in claim 1, wherein said step of heating comprises heating the glass sheet to the first predetermined temperature using radiant energy.

3. A method as claimed in claim 1 or 2, wherein said step of heating comprises heating the glass sheet to the first predetermined temperature from 482°C to 510°C (900°F to 950°F).

4. A method as claimed in any one of the preceding claims, wherein said step of heating comprises heating the glass sheet to the first predetermined temperature at a first predetermined rate.

5. A method as claimed in any one of the preceding claims, wherein the microwave energy is applied to the glass sheet when the glass sheet has reached at least the first predetermined temperature.

6. A method as claimed in any one of the preceding claims, wherein the microwave energy is applied to the glass sheet at a second predetermined rate.

7. A method as claimed in any one of the preceding claims, wherein the microwave energy is applied to the glass sheet to heat the glass sheet to the second predetermined temperature from 621°C (1150°F) to 676°C (1250°F).

8. A method as claimed in any one of the preceding claims, wherein said step of cooling comprises cooling the outer surface of the glass sheet simultaneously with said step of applying microwave energy.

9. A method as claimed in any one of the preceding claims, wherein said step of cooling comprises cooling the outer surface of the glass sheet to the third predetermined temperature of approximately 482°C (900°F).

10. A method as claimed in any one of the preceding claims, wherein said step of forming comprises moving the glass sheet through a plurality of forming rollers to bend the glass sheet to a desired curvature.

## Patentansprüche

1. Verfahren zum Erwärmen und Formen einer Glasscheibe, welches Verfahren folgende Schritte aufweist:
Erwärmen einer Glasscheibe bis auf wenigstens eine erste vorgegebene Temperatur;
Aufbringen von Mikrowellenenergie auf die Glasscheibe, so daß die Glasscheibe bis auf wenigstens eine zweite vorgegebene Temperatur aufgewärmt wird;
Abkühlen einer äußeren Fläche der Glasscheibe auf wenigstens eine dritte vorgegebene Temperatur; und
Formen der Glasscheibe zu einer vorgegebenen Gestalt unter Einsatz von Formwalzen.

2. Verfahren nach Anspruch 1, worin besagter Schritt der Erwärmung die Aufheizung der Glasscheibe bis auf die erste vorgegebene Temperatur unter Einsatz von Strahlungsenergie beinhaltet.

3. Verfahren nach Anspruch 1 oder 2, worin besagter Schritt der Erwärmung die Aufheizung der Glasscheibe bis auf die erste vorgegebene Temperatur von 482 °C bis 510 °C (900 °F bis 950 °F) beinhaltet.

4. Verfahren nach einem beliebigen der vorangehenden Ansprüche, worin besagter Schritt der Erwärmung die Aufheizung der Glasscheibe bis auf die erste vorgegebene Temperatur mit einer vorgegebenen Geschwindigkeit beinhaltet.

5. Verfahren nach einem beliebigen der vorangehenden Ansprüche, worin die Mikrowellenenergie erst auf die Glasscheibe aufgebracht wird, wenn die Glasscheibe wenigstens die erste vorgegebene Temperatur erreicht hat.

6. Verfahren nach einem beliebigen der vorangehenden Ansprüche, worin die Mikrowellenenergie mit einer zweiten vorgegebenen Geschwindigkeit auf die Glasscheibe aufgebracht wird.

7. Verfahren nach einem beliebigen der vorangehenden Ansprüche, worin die Mikrowellenenergie so auf die Glasscheibe aufgebracht wird, daß die Glasscheibe auf die zweite vorgegebene Temperatur von 621 °C (1150 °F) bis 676 °C (1250 °F) erwärmt wird.

8. Verfahren nach einem beliebigen der vorangehenden Ansprüche, worin besagter Schritt der Abkühlung die Abkühlung der äußeren Fläche der Glasscheibe gleichzeitig mit besagtem Schritt der Aufbringung von Mikrowellenenergie beinhaltet.

9. Verfahren nach einem beliebigen der vorangehenden Ansprüche, worin besagter Schritt der Abkühlung die Abkühlung der äußeren Fläche der Glasscheibe bis auf die dritte vorgegebene Temperatur von ungefähr 482 °C (900 °F) beinhaltet.

10. Verfahren nach einem beliebigen der vorangehenden Ansprüche, worin besagter Schritt der Formung beinhaltet, daß die Glasscheibe durch mehrere Formwalzen bewegt wird, so daß die Glasscheibe mit einer gewünschten Wölbung gewölbt wird.

## Revendications

1. Procédé de chauffage et de formage d'une feuille de verre, ledit procédé comprenant les étapes consistant à :
chauffer une feuille de verre jusqu'à au moins une première température prédéterminée,
appliquer une énergie hyperfréquence à la feuille de verre afin de chauffer la feuille de verre jusqu'à au moins une seconde température prédéterminée,
refroidir une surface extérieure de la feuille de verre jusqu'à au moins une troisième température prédéterminée, et
former la feuille de verre en utilisant des rouleaux de formage à une configuration prédéterminée.

2. Procédé selon la revendication 1, dans lequel ladite étape de chauffage comprend le chauffage de la feuille de verre jusqu'à la première température prédéterminée en utilisant une énergie rayonnante.

3. Procédé selon la revendication 1 ou 2, dans lequel ladite étape de chauffage comprend le chauffage de la feuille de verre jusqu'à la première température prédéterminée de 482 °C à 510 °C (900 °F à 950 °F).

4. Procédé selon l'une quelconque des revendications précédentes, dans lequel ladite étape de chauffage comprend le chauffage de la feuille de verre jusqu'à la première température prédéterminée à une première vitesse prédéterminée.

5. Procédé selon l'une quelconque des revendications précédentes, dans lequel l'énergie hyperfréquence est appliquée à la feuille de verre lorsque la feuille de verre a atteint au moins la première température prédéterminée.

6. Procédé selon l'une quelconque des revendications précédentes, dans lequel l'énergie hyperfréquence est appliquée à la feuille de verre à une seconde vitesse prédéterminée.

7. Procédé selon l'une quelconque des revendications précédentes, dans lequel l'énergie hyperfréquence est appliquée à la feuille de verre afin de chauffer la feuille de verre jusqu'à la seconde température prédéterminée de 621 °C (1 150 °F) à 676 °C (1 250 °F) :

8. Procédé selon l'une quelconque des revendications précédentes, dans lequel ladite étape de refroidissement comprend le refroidissement de la surface extérieure de la feuille de verre simultanément à ladite étape d'application d'énergie hyperfréquence.

9. Procédé selon l'une quelconque des revendications précédentes, dans lequel ladite étape de refroidissement comprend le refroidissement de la surface extérieure de la feuille de verre jusqu'à la troisième température prédéterminée d'approximativement 482 °C (900 °F).

10. Procédé selon l'une quelconque des revendications précédentes, dans lequel ladite étape de formage comprend le déplacement de la feuille de verre au travers d'une pluralité de rouleaux de formage afin de courber la feuille de verre à une courbure souhaitée.
